# EUROPEAN PATENT SPECIFICATION

(11) **EP 3 684 327 B1**
(45) Date of publication and mention of the grant of the patent: **19.05.2021**
(21) Application number: 18773436.3
(22) Date of filing: 20.09.2018
(51) Int. Cl.: A61K 8/19, A61K 8/46, A61K 8/42, A61Q 11/00

(54) **NOVEL COMPOSITION**
NEUARTIGE ZUSAMMENSETZUNG
NOUVELLE COMPOSITION

(30) Priority: 22.09.2017 GB 201715400
(43) Date of publication of application: 29.07.2020
(73) Proprietor: GlaxoSmithKline Consumer Healthcare (UK) IP Limited, Brentford, Middlesex TW8 9GS (GB)
(72) Inventor: HIPPALGOANKAR, Kanchan, Weybridge Surrey KT13 0DE (GB); PRIME, Constance Anne Marie, Weybridge Surrey KT13 0DE (GB)
(74) Representative: Lubienski, Michael John
(86) International application number: PCT/EP2018/075443
(87) International publication number: WO 2019/057809

(56) References cited:
- WO-A1-2006/052743
- WO-A1-2016/196131
- WO-A2-2013/020960

## Description

### FIELD OF THE INVENTION

The present invention relates to an aqueous sodium bicarbonate dentifrice composition comprising a novel surfactant system. In particular the invention relates to a dentifrice composition comprising sodium bicarbonate with a combination of surfactants comprising a betaine and sodium lauryl sulphate surfactant. Such a dentifrice composition demonstrates stability, good foam volume and pleasant organoleptic properties.

### BACKGROUND OF THE INVENTION

The use of sodium bicarbonate in dentifrices products is well known. One example is the commercial product parodontax which contains 67% (w/w) of sodium bicarbonate. Any dentifrice, in particular a sodium bicarbonate dentifrice must be acceptable from a consumer standpoint and demonstrate, for example, acceptable taste, consistency and adequate foaming on brushing of teeth. Any such composition must also be stable and not suffer from syneresis, which is the contraction of a gel accompanied by the separating out of liquid.

These organoleptic properties can be difficult to achieve, for example as described in WO2006/052743 (Church & Dwight Co., Inc.) which discloses an aqueous cleaning composition containing at least 20 wt% alkali metal bicarbonate and a surfactant comprising sodium decyl sulphate which is stated to offer advantageous properties over other surfactant systems investigated. Sodium decyl sulphate is claimed as the surfactant of choice as sodium lauryl sulphate (SLS) is rendered insoluble and provides very little foam when formulated in a bicarbonate containing dentifrice. In addition, sodium lauryl sarcosinate (SLOS), although producing significant foam, causes bicarbonate-containing dentifrices to undergo phase separation, even at room temperature. Moreover, combinations of SLS and SLOS could not produce a dentifrice composition that produces significant foam and remains phase stable at room temperature. Another surfactant approved for commercial use, cocamidopropyl betaine (CBT) was stated to produce significant foam in the presence of bicarbonate but caused bicarbonate-containing dentifrices to undergo phase separation, and combinations of SLS and CBT were found to be unable to produce a dentifrice composition that produces significant foam and remains stable.

It has now been found that the surfactant combination of SLS and a betaine in a dentifrice comprising at least 50% w/w sodium bicarbonate provides good foaming properties and remains phase stable by not exhibiting any syneresis. This is surprising in view of the teaching in WO2006/052743 that the combination of SLS and CBT (cocamidopropyl betaine) does not provide a bicarbonate dentifrice which is both phase stable and produces a significant amount of foam.

### SUMMARY OF THE INVENTION

The present invention provides a dentifrice composition comprising at least 50% w/w of sodium bicarbonate and 2% to 4% w/w of a surfactant system consisting of at least 0.3% w/w of a betaine in combination with at least 0.5% w/w of sodium lauryl sulphate (SLS).

### BRIEF DESCRIPTION OF THE DRAWINGS

Figure 1: Foam testing profile for toothpaste prototypes and control
Figure 2: In-house sensory study -foam level and consistency for toothpaste prototypes and control

### DETAILED DESCRIPTION OF THE INVENTION

Suitably the sodium bicarbonate is present in an amount from 55% to 90% w/w, preferably from 60% to 80% w/w, more preferably from 65% to 70% w/w; e.g. from 66% to 68% w/w.

The composition according to the invention comprises a surfactant system. The surfactant system consists of a betaine surfactant and sodium lauryl sulphate surfactant present in a total amount of 2% to 4% w/w, preferably in a total amount of 2% to 3% w/w.

Structurally, the betaine compounds contain an anionic functional group such as a carboxylate functional group and a cationic functional group such as quaternary nitrogen functional group separated by a methylene moiety. They include n-alkyl betaines such as cetyl betaine and behenyl betaine, and n-alkylamido betaines such as cocoamidopropyl betaine. In one embodiment the betaine is cocoamidopropyl betaine, commercially available from Evonik Industries AG under the trade name TEGO Betain C60.

Suitably the betaine is present in an amount ranging from 0.3% to 2% w/w, for example from 0.4% to 1.5% w/w and preferably from 0.5% to 0.7% w/w. A suitable betaine is TEGO Betain C60 which may be bought and formulated as a 47% aqueous solution and the figures quoted in brackets in the Examples are the active matter level of cocoamidopropyl betaine in the 47% solution.

Suitably the sodium lauryl sulphate (SLS) surfactant is present in an amount from 0.5 to 3.5% w/w, preferably in an amount from 1.0% to 3.0% w/w, more preferably in an amount from 1.5 to 2.5% w/w and most preferably in an amount from 1.75% to 2.25% w/w.

In addition to the above ingredients, compositions of the present invention may comprise a fluoride source, a desensitising agent, an anti-plaque agent, an anti-calculus agent, a whitening agent, an oral malodour agent, an anti-inflammatory agent, e.g. monoammonium glycyrrhizinate (MAG), an anti-microbial e.g. isopropylmethylphenol (IPMP), an anti-oxidant, an anti-fungal agent, a wound healing agent, e.g. hyaluronic acid, or a mixture of at least two thereof. Such agents may be included at levels to provide the desired therapeutic effect.

Suitable sources of fluoride ions for use in the compositions of the present invention include an alkali metal fluoride such as sodium fluoride, an alkali metal monofluorophosphate such a sodium monofluorophosphate, stannous fluoride, or an amine fluoride in an amount to provide from 25 to 3500pm of fluoride ions, preferably from 100 to 1500ppm. A typical fluoride source is sodium fluoride, for example the composition may contain 0.1 to 0.5% by weight of sodium fluoride, e.g. 0.204% by weight (equating to 927ppm of fluoride ions), 0.2542% by weight (equating to 1150ppm of fluoride ions) or 0.315% by weight (equating to 1426ppm of fluoride ions).

Such fluoride ions help promote the remineralisation of teeth and can increase the acid resistance of dental hard tissues for combating caries, dental erosion (i.e. acid wear) and/or tooth wear.

Sodium bicarbonate will provide abrasive properties to the composition however an additional abrasive may also be present, e.g. a silica that has high cleaning properties. Examples of high cleaning silica abrasives include those marketed as Zeodent 124, Tixosil 63, Sorbosil AC39, Sorbosil AC43 and Sorbosil AC35 and may be present in suitable amounts for example up to 20%, such as from 5 to 10%, preferably from 1.5 to 7%, e.g. 2% by weight of the total composition.

Suitable humectants for use in compositions of the invention include glycerin, xylitol, sorbitol, propylene glycol or polyethylene glycol, or mixtures of at least two thereof; which humectant may be present in the range from 1.0% to 20%, for example from 5% to 15% or from 7% to 10% by weight of the total composition.

Compositions of the present invention will contain additional formulating agents such as flavouring agents, sweetening agents, opacifying or colouring agents and preservatives, selected from those conventionally used in an oral hygiene composition art for such purposes.

Suitable sweetening agents include sodium saccharin and/or a natural product such as stevia which is a Steviol glycosides sweetener which is an extract from the Stevia plant and can be obtained from Tate & Lyle (known as TASTEVA Stevia sweetener) or Cargill (known as TRUVIA stevia leaf extract RA80).

Suitably the composition of the present invention includes a Steviol glycosides sweetener at levels of between 0.025 to 0.4% w/w, preferably between 0.05 and 0.2% w/w, more preferably from 0.075 to 0.125% w/w.

The composition of the present invention may be prepared by admixing the ingredients in the appropriate relative amounts in any order that is convenient and suitable for preparing a dentifrice product.

### Example 1 - Stability studies

The objectives of the study were to identify the formulation stability profile between formulations comprising combinations of SLS and Tego Betain or Adinol and Tego Betain.

The samples investigated were put on accelerated stability conditions at 40°C /75RH for 2 months and their physical appearance was monitored to investigate the products physical stability. The composition of the toothpastes was as follows.

**Table 1: Formulation details of prototypes 1 to 4**

| **Total level of surfactants % w/w** | **2.4%** | | **2.53 %** | |
|---|---|---|---|---|
| | **Prototype 1** | **Prototype 2** | **Prototype 3** | **Prototype 4** |
| **Ingredient** | **SLS/Tego %w/w** | **AD/Tego %w/w** | **SLS/Tego %w/w** | **AD/Tego %w/w** |
| Sodium Saccharin | 0.2500 | 0.2500 | 0.250 | 0.250 |
| Sodium Bicarbonate | 67.260 | 67.260 | 67.260 | 67.260 |
| Glycerol | 7.500 | 7.500 | 7.500 | 7.500 |
| Hydrated Silica | 2.000 | 2.000 | 2.000 | 2.000 |
| Titanium dioxide | 0.200 | 0.200 | 0.200 | 0.200 |
| Sicovit Red 30 | 0.0025 | 0.0025 | 0.0025 | 0.0025 |
| Xanthan Gum | 0.700 | 0.700 | 0.700 | 0.700 |
| Stevia | 0.100 | 0.100 | 0.100 | 0.100 |
| Sodium Fluoride | - | - | - | - |
| Topaz Optamint | 1.400 | 1.400 | 1.400 | 1.400 |
| 47% (aq) Tego Betain (Active Matter) | 1.277 (0.6) | 1.277 (0.6) | 1.134 (0.53) | 1.134 (0.53) |
| Adinol | - | 1.800 | - | 2.00 |
| Sodium Lauryl Sulphate | 1.800 | - | 2.00 | - |
| Purified Water | qs | qs | qs | qs |

Prototypes 1 and 3 are compositions of the present invention and Prototypes 2 and 4 are comparative examples.

### Results:

**Table 2: Stability results**

| **Formulation** | **2 Month Stability** |
|---|---|
| Prototype 1 | Pass (no syneresis) |
| Prototype 2 | Fail (syneresis) |
| Prototype 3 | Pass (no syneresis) |
| Prototype 4 | Fail (syneresis) |

### Conclusion:

The SLS/Tego formulations with total surfactant levels 2.4 and 2.53% w/w were stable for 2 months but the corresponding AD/Tego formulation were not stable and showed syneresis.

### Example 2 - Foam studies

The foam volumes of formulations comprising SLS alone (Prototype 5), Tego Betain alone (Prototype 7) and SLS with Tego Betain (Prototyes 6 and 8) were investigated. All Prototypes contained stevia. The control formulation is the commercial product parodontax Ultra Clean with 2.553% Tego Betain (1.2% active matter) and does not contain stevia.

### Testing

Foam properties were assessed using SITA Foam Testing R2000. The samples were prepared in a 1:9 ratio of paste to water, i.e. 100mg of sample paste was suspended in 900ml of water to form a slurry sample which was stirred for 90 minutes (750rpm). Samples were analysed in triplicate and 31 measurements were taken over a course of 60 mins.

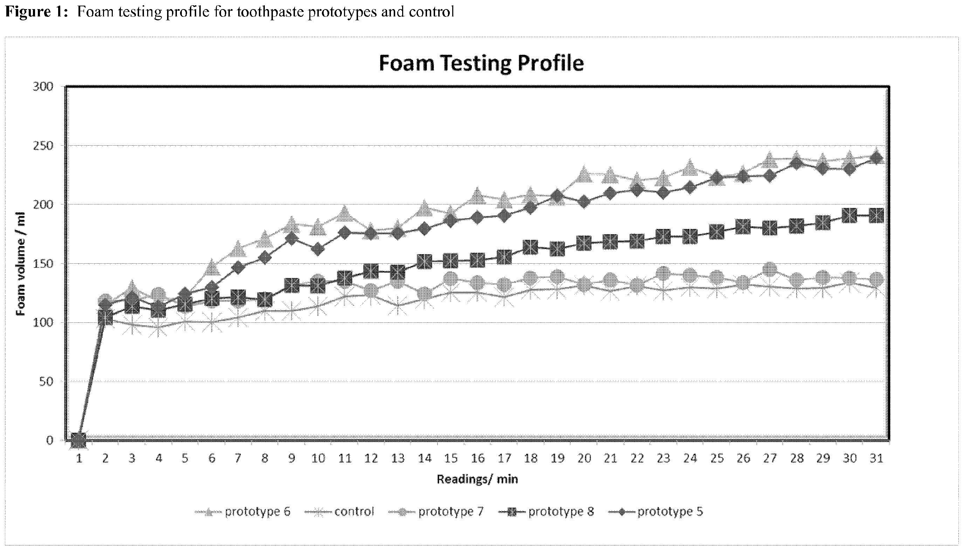

The following amounts of SLS, Tego Betain and TASTEVA Stevia sweetener were investigated. The results are shown in graphical representation as Figure 1 below.

**Table 3: Formulation details of prototypes 5 to 8**

| **Ingredient** | **Prototype 5 SLS** | **Prototype 6 SLS/Tego** | **Prototype 7 Tego** | **Prototype 8 SLS/Tego** |
|---|---|---|---|---|
| | %w/w | %w/w | %w/w | %w/w |
| Sodium Saccharin | 0.2500 | 0.2500 | 0.2500 | 0.2500 |
| Sodium Bicarbonate | 67.2600 | 67.2600 | 67.2600 | 67.2600 |
| Glycerol | 7.5000 | 7.5000 | 7.5000 | 7.5000 |
| Sodium Fluoride | - | 0.2210 | - | - |
| 47% (aq) Tego Betain solution (Active Matter) | - | 1.134 (0.533) | 2.553 (1.2) | 1.064 (0.5) |
| Sodium Lauryl Sulphate | 2.0 | 2.0 | - | 1.5 |
| Silica | 2.0 | 2.0 | 2.0 | 2.0 |
| Xanthan Gum | 0.70 | 0.70 | 0.70 | 0.70 |
| Stevia | 0.1 | 0.1 | 0.1 | 0.1 |
| Flavour | 1.400 | 1.400 | 1.400 | 1.400 |
| Sicovit Red 30 | 0.0025 | 0.0025 | 0.0025 | 0.0025 |
| Titanium Dioxide | 0.200 | 0.200 | 0.200 | 0.200 |
| Purified Water | qs | qs | qs | qs |

### Results

From the in vitro results in Figure 1 below it can be seen that prototype 6 (SLS/Tego Betain formulation of the invention) has a foam level that is similar to prototype 5 (SLS alone).

### Conclusion

The foaming properties of prototype 7 (Tego Betain only formulation) was slightly enhanced by the addition of stevia sweetener when compared with the Tego Betaine control without stevia. Prototypes 6 and 8 (SLS/Tego Betain formulations of the invention) also with stevia showed good foaming levels, especially prototype 6 which has SLS present at a level of 2.0% w/w together with the Tego Betain surfactant.

### Example 3 - Further foam studies

Further foam levels and foam consistency studies of prototype 5, prototype 6 and prototype 7 were investigated along with the Control formulation (as above).

### Testing

Panellists received about 1.5g of toothpaste dispensed on a brush then dipped the toothbrush in water. They brushed their teeth for 1 timed minute without swallowing and spat the residue.

The panellists assessed the defined sensory attributes immediately after spitting; they then rinsed swirling around with 30 ml of water once and spat again. Foam level and foam consistency were evaluated and each was conducted in triplicate.

### Results

From the in vivo results it can be seen that prototype 6 (SLS / Tego Betaine combination) showed better foam consistency than either prototype 5 (SLS alone) and prototype 7 (Tego Betaine alone) and the Control but with regard to foam level, it was in parity with the Control. The Control showed significantly better foaming level and consistency than the prototype 7 formulation.

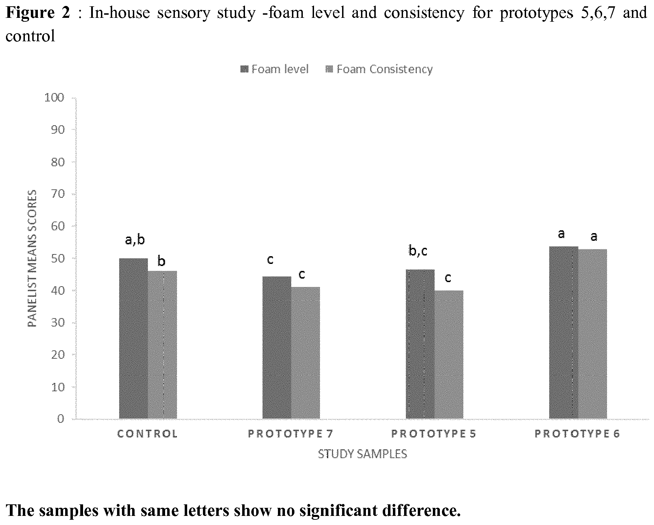

The results are also shown in graphical representation as Fig 2 below. Foam levels are shown in the first column of each sample and Foam consistency are shown in the second column.

### Conclusion

There is an organoleptic benefit in selecting prototype 6 (SLS / Tego Betaine combination) as a detergent system in a toothpaste formulation versus prototype 7 (Tego Betain alone), prototype 5 (SLS alone) or the Control formulation. The consumer will perceive more foam and a thicker texture during brushing.

### Example 4 - Further formulations of the invention

**Table 4: Additional formulations of the invention**

| **Ingredient** | **%w/w** | **%w/w** |
|---|---|---|
| Sodium Bicarbonate | 67.260 | 67.260 |
| Glycerin 95% | 7.8947 | 7.8947 |
| Hydrated Silica | 2.000 | 2.000 |
| Titanium dioxide | 0.500 | 0.500 |
| Xanthan Gum | 0.700 | 0.440 |
| Stevia | 0.100 | 0.100 |
| Sodium Fluoride | 0.205 | 0.205 |
| Cocamidopropyl Betaine TB CK D (active matter) | 1.134 (0.533) | 1.134 (0.533) |
| Sodium Lauryl Sulphate | 2.000 | 2.000 |
| Sodium Saccharin | 0.350 | 0.350 |
| Monoammonium Glycyrrhizinate | 0.100 | 0.100 |
| Isopropylmethylphenol | 0.105 | 0.105 |
| Flavour | 1.300 | 1.300 |
| Red No. 227 | 0.0005 | 0.0005 |
| Yellow Ferric Oxide | 0.005 | 0.005 |
| Purified Water | qs | qs |

## Claims

1. A dentifrice composition comprising at least 50% w/w of sodium bicarbonate and 2% to 4% w/w of a surfactant system consisting of at least 0.3% w/w of a betaine in combination with at least 0.5% w/w of sodium lauryl sulphate (SLS).

2. A composition according to claim 1 wherein the betaine is cocoamidopropyl betaine.

3. A composition according to claim 1 to 2 wherein the betaine is present in an amount from 0.3 to 2.0% w/w.

4. A composition according to any one of claims 1 to 3 wherein the SLS is present in an amount 0.5 to 3.5% w/w.

5. A composition according to any one of claims 1 to 4 wherein the sodium bicarbonate is present in an amount from 55 to 90% by weight of the composition.

6. A composition according to any one of the above claims comprising stevia sweetener.

7. A composition according to claim 6 wherein the stevia is present in an amount from 0.025 to 0.4% w/w.

8. A composition according to any one of claims 1 to 7 additionally comprising a fluoride source, a desensitizing agent, an anti-plaque agent, an anti-calculus agent, a whitening agent, an oral malodour agent, an anti-inflammatory agent, an antimicrobial agent, an anti-oxidant, an anti-fungal agent, a wound healing agent or a mixture of at least two thereof.

## Patentansprüche

1. Zahnputzmittelzusammensetzung, umfassend mindestens 50 Gew.-% Natriumbicarbonat und 2 bis 4 Gew.-% eines Tensidsystems, bestehend aus mindestens 0,3 Gew.-% eines Betains in Kombination mit mindestens 0,5 Gew.-% Natriumlaurylsulfat (SLS).

2. Zusammensetzung gemäß Anspruch 1, wobei das Betain Cocoamidopropylbetain ist.

3. Zusammensetzung gemäß Anspruch 1 bis 2, wobei das Betain in einer Menge von 0,3 bis 2,0 Gew.-% vorhanden ist.

4. Zusammensetzung gemäß irgendeinem der Ansprüche 1 bis 3, wobei das SLS in einer Menge von 0,5 bis 3,5 Gew.-% vorhanden ist.

5. Zusammensetzung gemäß irgendeinem der Ansprüche 1 bis 4, wobei das Natriumbicarbonat in einer Menge von 55 bis 90 Gew.-% der Zusammensetzung vorhanden ist.

6. Zusammensetzung gemäß irgendeinem der obigen Ansprüche, umfassend Stevia-Süßstoff.

7. Zusammensetzung gemäß Anspruch 6, wobei das Stevia in einer Menge von 0,025 bis 0,4 Gew.-% vorhanden ist.

8. Zusammensetzung gemäß irgendeinem der Ansprüche 1 bis 7, zusätzlich umfassend eine Fluoridquelle, ein desensibilisierendes Mittel, ein Anti-Zahnbelagmittel, ein Anti-Zahnsteinmittel, ein Aufhellungsmittel, ein Mittel gegen Mundgeruch, ein entzündungshemmendes Mittel, ein Antimikrobiotikum, ein Antioxidans, ein Antipilzmittel, ein Wundheilungsmittel oder eine Mischung aus mindestens zwei von diesen.

## Revendications

1. Composition de dentifrice comprenant au moins 50% p/p de bicarbonate de sodium et 2% à 4% p/p d'un système tensioactif consistant en au moins 0,3% p/p d'une bétaïne en combinaison avec au moins 0,5% p/p de laurylsulfate de sodium (SLS).

2. Composition selon la revendication 1 dans laquelle la bétaïne est la cocoamidopropylbétaïne.

3. Composition selon la revendication 1 à 2 dans laquelle la bétaïne est présente en une quantité de 0,3 à 2,0% p/p.

4. Composition selon l'une quelconque des revendications 1 à 3 dans laquelle le SLS est présent en une quantité de 0,5 à 3,5% p/p.

5. Composition selon l'une quelconque des revendications 1 à 4 dans laquelle le bicarbonate de sodium est présent en une quantité de 55 à 90% en poids de la composition.

6. Composition selon l'une quelconque des revendications précédentes comprenant un édulcorant stévia.

7. Composition selon la revendication 6 dans laquelle la stévia est présente en une quantité de 0,025 à 0,4% p/p.

8. Composition selon l'une quelconque des revendications 1 à 7 comprenant en outre une source de fluorure, un agent désensibilisant, un agent anti-plaque, un agent anti-tartre, un agent blanchissant, un agent pour la mauvaise haleine oral, un agent anti-inflammatoire, un agent antimicrobien, un antioxydant, un agent antifongique, un agent cicatrisant ou un mélange d'au moins deux de ceux-ci.
